Europäisches Patentamt

European Patent Office

Office européen des brevets

(19)

(11) Veröffentlichungsnummer : **0 325 979 B1**

# EUROPÄISCHE PATENTSCHRIFT

(12)

(45) Veröffentlichungstag der Patentschrift :
**04.12.91 Patentblatt 91/49**

(51) Int. Cl.$^5$ : **C07C 37/04, C07C 39/15**

(21) Anmeldenummer : **89100640.5**

(22) Anmeldetag : **14.01.89**

(54) **Verfahren zur Herstellung von 4,4'-Dihydroxybiphenyl.**

Die Akte enthält technische Angaben, die nach dem Eingang der Anmeldung eingereicht wurden und die nicht in dieser Patentschrift enthalten sind.

(30) Priorität : **23.01.88 DE 3801943**

(43) Veröffentlichungstag der Anmeldung :
**02.08.89 Patentblatt 89/31**

(45) Bekanntmachung des Hinweises auf die Patenterteilung :
**04.12.91 Patentblatt 91/49**

(84) Benannte Vertragsstaaten :
**CH DE FR GB IT LI**

(56) Entgegenhaltungen :
**EP-A- 0 085 883**
**EP-A- 0 092 772**
**DE-A- 3 226 391**
**DE-C- 810 871**
**DE-C- 815 645**
**US-A- 4 243 822**

(73) Patentinhaber : **BASF Aktiengesellschaft**
**Carl-Bosch-Strasse 38**
**W-6700 Ludwigshafen (DE)**

(72) Erfinder : **Eichenauer, Ulrich, Dr.**
**Paul-Ehrlich-Strasse 25 a**
**W-6000 Frankfurt 70 (DE)**
Erfinder : **Neumann, Peter, Dr.**
**Poststrasse 28**
**W-6800 Mannheim 31 (DE)**

## Beschreibung

Die vorliegende Erfindung betrifft ein neues Verfahren zur Herstellung von 4,4'-Dihydroxybiphenyl durch Behandlung von Biphenyl-4,4'-disulfonsäure oder deren Alkalisalze in einer Alkalischmelze bei einer Temperatur von 280 bis 360°C, wobei die Schmelze Kaliumhydroxid, gegebenenfalls Natriumhydroxid sowie ein Alkalicarbonat enthält.

Die Herstellung von 4,4'-Dihydroxybiphenyl durch Alkalischmelze der entsprechenden Disulfonsäure ist bekannt. Sie wird meistens unter Druck in Gegenwart von Wasser (DE-A-3204079, EP-A-85883) oder von hochsiedenden Chlorkohlenwasserstoffen (EP-A-92772) durchgeführt. Dabei ist aber ein hoher apparativer Aufwand erforderlich.

Schmelzreaktionen, die bei atmosphärischem Druck vorgenommen werden, sind ebenfalls bekannt. Beispielsweise ist in J. Chem. Soc. Japan, Band 84, S. 143, 1963, die Umsetzung von 1 Mol des Dikaliumsalzes der Biphenyl-4,4'-disulfonsäure mit 12,5 Mol Kaliumhydroxid und 8,9 Mol Natriumhydroxid bei einer Temperatur von 330°C beschrieben. Das Zielprodukt fällt dabei nach der Reinigung in einer Ausbeute von 19% an.

Die US-A-4243822 beschreibt die Verwendung von 12,5 Mol Kaliumhydroxid pro Mol Dikaliumsalz der Biphenyl-4,4'-disulfonsäure bei einer Temperatur von 335 bis 340°C. Nachteilig ist hierbei der große Überschuß an teurem Kaliumhydroxid.

Nach der Lehre der JP-A-112844/1979 soll die Umsetzung in reinem Natriumhydroxid vorgenommen werden. Diese Reaktion bedarf einer Temperatur von 380°C, die jedoch mit konventionellen Wärmeübertragungsmedien nicht mehr erreichbar ist.

Ein genereller Nachteil dieser Alkalihydroxidschmelzen ist ihr Aufschäumen, das bei der hohen Viskosität der Schmelze durch das bei der Reaktion entstehende Wasser verursacht wird. Zur Erniedrigung der Viskosität von Alkalischmelzen wurden Zusätze, wie Natriumacetat (JP-A-8720/1960) oder Kohle, Alkalisulfite, Alkalisulfate oder Erdalkalihydroxide (DE-B-815645) vorgeschlagen.

Aus der DE-A-3226391 ist die Umsetzung von 1 Mol des Dikaliumsalzes der Biphenyl-4,4'-disulfonsäure mit 8 Mol Kaliumhydroxid in Gegenwart von 0,75 Mol Kaliumsulfat bekannt. Die Reaktion wird dabei in einer Kugelmühle durchgeführt.

Schließlich beschreibt die DE-C-810871 die Umsetzung von Arylsulfonaten mit Calciumhydroxid, wobei vorgeschlagen wird, die Reaktion zusätzlich in Gegenwart u.a. von Natrium- oder Kaliumcarbonat durchzuführen.

Bei den Verfahren des standes der Technik wird allerdings das Problem des Aufschäumens nicht gelöst.

Aufgabe der vorliegenden Erfindung war es deshalb, ein Verfahren zur Herstellung von 4,4'-Dihydroxybiphenyl durch Alkalischmelze von Biphenyl-4,4'-disulfonsäure oder deren Alkalisalze bereitzustellen, das ohne großen technischen Aufwand durchgeführt werden kann und bei dem das Aufschäumen der Alkalischmelze unterdrückt wird.

Es wurde nun gefunden, daß die Herstellung von 4,4'-Dihydroxybiphenyl durch Behandlung von Biphenyl-4,4'-disulfonsäure oder deren Alkalisalzen in einer Alkalihydroxid-Schmelze, vorteilhaft gelingt, wenn man die Behandlung in einer Schmelze, die 7 bis 14 Mol Kaliumhydroxid und 0 bis 8 Mol Natriumhydroxid, jeweils bezogen auf 1 Mol Biphenyl-4,4'-disulfonsäure, enthält und einen Wassergehalt von 10 bis 20 Gew.-%, bezogen auf das Gesamtgewicht der Schmelze, aufweist, in Gegenwart eines Alkalicarbonats bei einer Temperatur von 280 bis 360°C durchführt.

Beim erfindungsgemäßen verfahren können neben der Biphenyl-4,4'-disulfonsäure auch deren Dialkalisalze, z.B. die Dilithium-, Dinatrium- oder Dikaliumsalze, umgesetzt werden.

Die Herstellung der Ausgangsmaterialien ist bekannt und erfolgt beispielsweise durch Sulfonieren von Biphenyl mit Oleum, Schwefelsäure oder Chlorsulfonsäure (siehe z.B. J. Chem. Soc., Perkin II, S. 1560, 1977). Die Alkalisulfonate können aus den Sulfoniergemischen durch Aussalzen (Helv. Chem. Acta 14, S. 751, 1931) oder Neutralisation hergestellt werden.

Das erfindungsgemäße Verfahren wird zweckmäßig so durchgeführt, daß man eine Schmelze, die 7 bis 14 Mol Kaliumhydroxid und 0 bis 8 Mol Natriumhydroxid, vorzugsweise 7 bis 14 Mol Kaliumhydroxid und 0 bis 4 Mol Natriumhydroxid, jeweils bezogen auf 1 Mol Biphenyl-4,4'-disulfonsäure, und ein Alkalicarbonat enthält, bereitet.

Als Alkalicarbonate kommen z.B. Lithium-, Natrium- oder Kaliumcarbonat in Betracht. Vorzugsweise verwendet man 10 bis 100 Mol-%, insbesondere 50 bis 100 Mol-% Alkalicarbonat, jeweils bezogen auf 1 Mol Biphenyl-4,4'-disulfonsäure. Die Verwendung von Natriumcarbonat sei besonders hervorgehoben.

Die Schmelze wird auf eine Temperatur von 280 bis 360°C, vorzugsweise 300 bis 340°C und insbesondere ca. 320°C erhitzt.

Unter Einhaltung der erfindungsgemäßen Temperatur werden nun die Biphenyl-4,4'-disulfonsäure oder deren Dialkalisalze zur Schmelze dosiert. Die Dosierung kann dabei in Substanz, in wäßriger Lösung oder in shwefelsaurer Lösung erfolgen.

Bevorzugt dosiert man eine 20 bis 50 gew.%ige wäßrige Lösung der Biphenyl-4,4'-disulfonsäure, die eine Temperatur von 20 bis 100°C, vorzugsweise 40 bis 100°C aufweist, zur Schmelze.

Weiterhin bevorzugt ist die Zugabe einer Lösung von Biphenyl-4,4'-disulfonsäure in 30 bis 70 gew.%iger Schwefelsäure zur Schmelze. Die Konzentration der Lösung an Biphenyl-4,4'-disulfonsäure beträgt dabei 20 bis 50 Gew.%, bezogen auf das Gewicht der Lösung, ihre Temperatur 20 bis 100°C, vorzugsweise 40 bis 100°C.

Weiterhin bevorzugt ist die Zugabe eines Dialkalisalzes der Biphenyl-4,4'-disulfonsäure in Substanz.

Mit Ausnahme des letztgenannten Falls wird dabei das in die Schmelze eingebrachte und/oder bei der Neutralisationsreaktion entstehende Wasser abdestilliert.

Während der Reaktion, die im allgemeinen 2 bis 6 Stunden in Anspruch nimmt, weist die Schmelze in der Regel einen Wassergehalt von 10 bis 20 Gew.%, vorzugsweise ca. 15 Gew.%, jeweils bezogen auf das Gesamtgewicht der Schmelze, auf. Der Wassergehalt kann dabei durch Rückführung eines Teils des abdestillierten Wassers eingestellt werden.

Nach beendeter Umsetzung wird das Reaktionsgemisch durch Zudosieren von Wasser und gleichzeitiger Erniedrigung der Temperatur der Schmelze (z.B. durch Erniedrigung der Heizbadtemperatur) abgekühlt. Dabei ist zu beachten, daß die Siedetemperatur des Gemisches nicht überschritten wird. Das so mit Wasser verdünnte Reaktionsgemisch wird bei einer Temperatur von ca. 90°C mit konzentrierter Salzsäure bis zu einem pH-Wert von 6,5 bis 6,7 versetzt und das dabei freigesetzte 4,4'-Dihydroxybiphenyl mittels Filtration gewonnen.

Die Reinigung des als Filterrückstand anfallenden rohen 4,4'-Dihydroxybiphenyl kann durch Extraktion geschehen. Geeignete Extraktionsmittel sind z.B. Alkohole, wie Methanol, Ethanol oder Isopropanol, Ketone, wie Aceton oder Methylethylketon, oder Ester, wie Essigsäureethylester.

Das erfindungsgemäße Verfahren liefert 4,4'-Dihydroxybiphenyl in guter Ausbeute und hoher Reinheit. Durch die Anwesenheit von Alkalicarbonat wird die angesprochene Schaumbildung, die für ein im technischen Maßstab durchgeführtes Verfahren ein Sicherheitsrisiko darstellt, überraschenderweise unterdrückt.

4,4'-Dihydroxybiphenyl ist ein wertvolles Zwischenprodukt. Es dient z.B als Monomer-Bestandteil für Polyester oder wird für die Herstellung von photographischen Entwicklern benötigt.

Die folgenden Beispiele sollen die Erfindung näher erläutern.

Beispiel 1

In einem 1 l Rührkessel aus V2A-Stahl wurden 400 g (6,1 Mol) Kaliumhydroxid (Wassergehalt 15 Gew.%) und 40 g (0,38 Mol) Natriumcarbonat vorgelegt und 195 g (0,5 Mol) des Dikaliumsalzes der Biphenyl-4,4'-disulfonsäure bei 280°C eingetragen. Man erhöhte die Temperatur dann auf 320°C, hielt bei dieser Temperatur drei Stunden und kühlte die Schmelze durch konstantes Zudosieren von Wasser und gleichzeitiger Erniedrigung der Heizbadtemperatur ab. Dann verdünnte man das Reaktionsgemisch mit Wasser und säuerte bei 90°C mit konzentrierter Salzsäure bis zu einem pH-Wert von 6,5 an. Man saugte den entstandenen Niederschlag ab und extrahierte 4,4'-Dihydroxybiphenyl mittels Aceton. Nach dem Entfernen des Extraktionsmittels erhielt man 85,5 g (92%) 4,4'-Dihydroxybiphenyl, das eine Reinheit von > 99% (HPIC) aufwies.

Die folgenden Beispiele wurden analog durchgeführt. Anstelle von Kaliumhydroxid wurde jedoch jeweils eine Mischung aus Kaliumhydroxid und Natriumhydroxid verwendet.

| | Alkalihydroxid [Mol] | | | |
|---|---|---|---|---|
| Bsp. Nr. | KOH | NaOH | Ausbeute [%] | Reinheit[%] |
| 2 | 5,2 | 1,5 | 86 | 99,6 |
| 3 | 4,6 | 2,5 | 80 | 99,7 |

**Patentansprüche**

1. Verfahren zur Herstellung von 4,4'-Dihydroxybiphenyl durch Behandlung von Biphenyl-4,4'-disulfonsäure oder deren Alkalisalzen in einer Alkalihydroxid-Schmelze, dadurch gekennzeichnet, daß man die Behandlung in einer Schmelze, die 7 bis 14 Mol Kaliumhydroxid und 0 bis 8 Mol Natriumhydroxid, jeweils bezogen auf 1 Mol Biphenyl-4,4'-disulfonsäure, enthält und einen Wassergehalt von 10 bis 20 Gew.-%, bezogen auf das Gesamtgewicht der Schmelze, aufweist, in Gegenwart eines Alkalicarbonats bei einer Temperatur von 280 bis 360°C durchführt.

2. Verfahren gemäß Anspruch 1, dadurch gekennzeichnet, daß man die Behandlung bei einer Temperatur von 300 bis 340°C durchführt.

3. Verfahren gemäß Anspruch 1, dadurch gekennzeichnet, daß man die Behandlung in Gegenwart von 10 bis 100 Mol% eines Alkalicarbonats, bezogen auf 1 Mol Biphenyl-4,4'-disulfonsäure, durchführt.

4. Verfahren gemäß Anspruch 1, dadurch gekennzeichnet, daß man die Behandlung in Gegenwart von Natriumcarbonat durchführt.

5. Verfahren gemäß Anspruch 1, dadurch gekennzeichnet, daß man die Behandlung in einer Schmelze, die 7 bis 14 Mol Kaliumhydroxid und 0 bis 4 Mol Natriumhydroxid, jeweils bezogen auf 1 Mol Biphenyl-4,4-disulfonsäure, enthält, durchführt.

## Claims

1. A process for the preparation of 4,4'-dihydroxybiphenyl by treating biphenyl-4,4'-disulfonic acid or one of its alkali metal salts in an alkali metal hydroxide melt, wherein the treatment is carried out in a melt which contains from 7 to 14 moles of potassium hydroxide and from 0 to 8 moles of sodium hydroxide, based in each case on 1 mole of biphenyl-4,4'-disulfonic acid, and has a water content of from 10 to 20% by weight, based on the total weight of the melt, in the presence of an alkali metal carbonate at from 280 to 360°C.

2. A process as claimed in claim 1, wherein the treatment is carried out at from 300 to 340°C.

3. A process as claimed in claim 1, wherein the treatment is carried out in the presence of from 10 to 100 mol % of an alkali metal carbonate per mole of biphenyl-4,4'-disulfonic acid.

4. A process as claimed in claim 1, wherein the treatment is carried out in the presence of sodium carbonate.

5. A process as claimed in claim 1, wherein the treatment is carried out in a melt which contains from 7 to 14 moles of potassium hydroxide and from 0 to 4 moles of sodium hydroxide, based in each case on 1 mole of biphenyl-4,4'-disulfonic acid.

## Revendications

1. Procédé de préparation de 4,4'-dihydroxybiphényle par traitement d'acide biphényl-4,4'-disulfonique ou de sels alcalins dans une masse fondue d'hydroxyde alcalin, caractérisé par le fait que l'on effectue le traitement en présence d'un carbonate alcalin, à une température de 280 à 360 degrés C, dans une masse fondue qui contient, par mole d'acide biphényl-4,4'-disulfonique, 7 à 14 moles d'hydroxyde de potassium et 0 à 8 moles d'hydroxyde de sodium, et présente, rapporté au poids total de la masse fondue, une teneur en eau comprise entre 10 et 20% en poids.

2. Procédé selon la revendication 1, caractérisé par le fait que l'on effectue la traitement à une température de 300 à 340 degrés C.

3. Procédé selon la revendication 1, caractérisé par le fait que l'on effectue le traitement en présence de 10 à 100% (moles) d'un carbonate alcalin par mole d'acide biphényl-4,4'-disulfonique.

4. Procédé selon la revendication 1, caractérisé par le fait que l'on effectue le traitement en présence de carbonate de sodium.

5. Procédé selon la revendication 1, caractérisé par le fait que l'on effectue le traitement dans une masse fondue contenant, par mole d'acide biphényl-4,4'-disulfonique, 7 à 14 moles d'hydroxyde de potassium et 0 à 4 moles d'hydroxyde de sodium.